# EUROPEAN PATENT SPECIFICATION

(11) **EP 4 028 110 B1**
(45) Date of publication and mention of the grant of the patent: **24.06.2026**
(21) Application number: 19783788.3
(22) Date of filing: 23.09.2019
(51) Int. Cl.: A61M 39/10, A61M 39/12, A61M 5/14, A61M 25/02, A61M 25/00, A61M 5/158, A61M 39/06, A61M 39/02, A61M 1/36

(54) **VASCULAR ACCESS SYSTEMS AND METHODS THEREOF**
VASKULÄRE ZUGANGSSYSTEME UND VERFAHREN DAFÜR
SYSTÈMES D'ACCÈS VASCULAIRE ET PROCÉDÉS CORRESPONDANTS

(43) Date of publication of application: 20.07.2022
(73) Proprietor: Bard Peripheral Vascular, Inc., Franklin Lakes, NJ 07417 (US)
(72) Inventor: GOOCH, Nathan, Centerville, UT 84014 (US); FORSYTH, Bradley, Sandy, UT 84093 (US)
(74) Representative: Hoffmann Eitle
(86) International application number: PCT/US2019/052477
(87) International publication number: WO 2021/061095

(56) References cited:
- US-A1- 2010 121 313
- US-A1- 2013 046 224
- US-A1- 2014 024 998
- US-A1- 2017 143 890
- US-A1- 2018 078 751
- US-A1- 2019 070 399

## Description

### BACKGROUND

A non-cuffed hemodialysis catheter is often used in an emergency or for a relatively short period of time (e.g., < 3 weeks) when a patient is in immediate need of hemodialysis due to kidney failure. Likewise, a cuffed hemodialysis catheter is often used when a patient is in immediate need of hemodialysis, but the cuffed hemodialysis catheter can be used for a relatively long period of time (e.g., > 3 weeks). Both types of hemodialysis catheter are typically used until permanent vascular access can be established by way of an arteriovenous ("AV") fistula or graft, each of which require surgery and recovery time. Once placed, such hemodialysis catheters have an extracorporeal portion that requires routine maintenance to avoid clots and infection. In addition, there is a risk of the patient or others tampering with the extracorporeal portion of such hemodialysis catheters, which can lead to air embolism. In view of the foregoing, there is a need for vascular access systems and methods thereof that obviate the routine maintenance and the risk of tampering inherent in existing hemodialysis catheters.

Disclosed herein are vascular access systems and methods thereof that address at least the foregoing need.

US 2019/070399 discloses an infusion needle assembly 60 comprising, on an infusion side, a single needle 62 to puncture a septum 23 and, on an aspiration side, two needles 64 to puncture a septum 25. US 2018/078751 discloses various different low-profile access ports for subcutaneous implantation within the body of a patient.

US 2017/0143890 discloses a dual lumen retrograde catheter for a dialysis treatment comprising a hub allowing for communication between luer connectors and cannulas, respectively.

### SUMMARY

In order to achieve the above objects and effects, the technical solution implemented by the present invention is defined in the independent claim 1.

According to the invention, a vascular access system includes, in some embodiments, a port and a single-use access device. The port is configured for subdermal implantation. The port includes a port hub and a catheter tube. The port hub has an access funnel and a septum defining a pair of port-hub lumens. The catheter tube has a septum defining a pair of catheter-tube lumens fluidly coupled to the pair of port-hub lumens. The access device includes a bifurcated hub and a pair of cannulas. The bifurcated hub includes a pair of access device-hub lumens. The pair of cannulas defines a pair of cannula lumens fluidly coupled to the pair of access device-hub lumens. The pair of cannulas is configured to simultaneously insert into the access funnel and fluidly couple the pair of cannula lumens to the pair of catheter-tube lumens when the pair of cannulas is fully seated in the port hub.

In some embodiments, the pair of cannulas is configured to pierce skin of a patient before inserting the pair of cannulas into the access funnel of the port when implanted under the skin.

In some embodiments, the single-use access device includes a pair of extension legs defining a pair of extension-leg lumens fluidly coupled to the pair of access device-hub lumens.

In some embodiments, the port hub has a pair of valves in a distal-end portion of the access funnel configured to open when the pair of cannulas is inserted into the pair of valves.

In some embodiments, the port hub has a bifurcated stem extending from the port hub configured to insert into the pair of catheter-tube lumens.

In some embodiments, the port includes a catheter lock over the catheter tube over the bifurcated stem. The catheter lock is configured to lock the catheter tube onto the bifurcated stem.

In some embodiments, the vascular access system is configured for continuous flow at a flow rate of at least 300 to 500 mL/min.

Also disclosed herein is a port (not according to the wording of the claims) configured for subdermal implantation including, in some embodiments, a port hub and a catheter tube. The port hub has an access funnel and a septum defining a pair of port-hub lumens. The catheter tube has a septum defining a pair of catheter-tube lumens fluidly coupled to the pair of port-hub lumens.

In some embodiments, the port hub has a pair of valves in a distal-end portion of the access funnel configured to open when a pair of cannulas of a single-use access device is inserted into the pair of valves.

In some embodiments, the port hub has a bifurcated stem extending from the port hub configured to insert into the pair of catheter-tube lumens.

In some embodiments, the port includes a catheter lock over the catheter tube over the bifurcated stem. The catheter lock is configured to lock the catheter tube onto the bifurcated stem.

In some embodiments, the catheter tube is a radiopaque polyurethane catheter tube.

In some embodiments, the access funnel is titanium or an alloy thereof. The access funnel is configured for a relatively low-angle approach by a single-use access device.

In some embodiments, the access funnel is disposed within a silicone body of the port hub.

Also disclosed herein is a single-use access device (not according to the wording of the claims) including, in some embodiments, a bifurcated hub and a pair of cannulas. The bifurcated hub includes a pair of access device-hub lumens. The pair of cannulas define a pair of cannula lumens fluidly coupled to the pair of access device-hub lumens. The pair of cannulas is configured to simultaneously insert into an access funnel of an implantable port and fluidly couple the pair of cannula lumens to a pair of catheter-tube lumens when the pair of cannulas is fully seated in the implantable port.

In some embodiments, the pair of cannulas is configured to pierce skin of a patient before inserting the pair of cannulas into the access funnel of the port when implanted under the skin.

In some embodiments, the access device further includes a pair of extension legs defining a pair of extension-leg lumens fluidly coupled to the pair of access device-hub lumens.

In some embodiments, the access device further includes a pair of Luer connectors. Each Luer connector is connected to an extension leg of the pair of extension legs.

Also disclosed herein is a method (not according to the wording of the claims) of vascular access system including, in some embodiments, a step of locating an access site and identifying an implanted port implanted in a patient at the access site by palpation of the patient. The port includes a port hub and a catheter tube. The port hub has an access funnel and a septum defining a pair of port-hub lumens. The catheter tube has a septum defining a pair of catheter-tube lumens fluidly coupled to the pair of port-hub lumens. The method also includes a step of cleaning and disinfecting the access site with an antiseptic for skin disinfection. The method also includes a step of preparing a single-use access device including priming the access device with saline. The access device includes a bifurcated hub and a pair of cannulas. The bifurcated hub includes a pair of access device-hub lumens. The pair of cannulas defines a pair of cannula lumens fluidly coupled to the pair of access device-hub lumens. The method also includes a step of stabilizing the implanted port with a non-dominant, sterile-gloved hand. The method also includes a step of simultaneously inserting the pair of cannulas into the access funnel until the pair of cannulas is fully seated in the port hub, thereby fluidly coupling the pair of cannula lumens to the pair of catheter-tube lumens.

In some embodiments, inserting the pair of cannulas into the access funnel includes piercing skin of the patient and inserting the pair of cannulas into the access funnel with a shallow angle of approach of less than about 30° between the patient and the pair of cannulas.

In some embodiments, the method further includes a step of aspirating the vascular access system for blood flow. The method also includes a step of securely dressing the access site with a wound dressing.

These and other features of the concepts provided herein will become more apparent to those of skill in the art in view of the accompanying drawings and following description, which describe particular embodiments of such concepts in greater detail.

### DRAWINGS

FIG. 1 illustrates a vascular access system including a port and an access device disposed in the port in accordance with some embodiments.
FIG. 2 illustrates the vascular access system of FIG. 1 with the access device separated from the port in accordance with some embodiments.
FIG. 3 illustrates a cross-section of a port hub of the port in accordance with some embodiments.
FIG. 4 illustrates an exploded view of the port hub in accordance with some embodiments.
FIG. 5 illustrates a close-up view of a distal-end portion of the port hub in accordance with some embodiments.
FIG. 6 illustrates a cross-section of a catheter tube of the port in accordance with some embodiments.
FIG. 7 illustrates a detailed view of a pair of cannulas of the access device in accordance with some embodiments.
FIG. 8 illustrates the port implanted in a patient in accordance with some embodiments.

### DESCRIPTION

Regarding terms used herein, it should also be understood the terms are for the purpose of describing some particular embodiments. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

Unless defined otherwise, all technical and scientific terms used herein have the same meaning as commonly understood by those of ordinary skill in the art.

As set forth above, there is a need for vascular access systems and methods thereof that obviate the routine maintenance and the risk of tampering inherent in existing hemodialysis catheters. Disclosed herein are vascular access systems and methods thereof that address at least the foregoing need.

### Vascular access systems

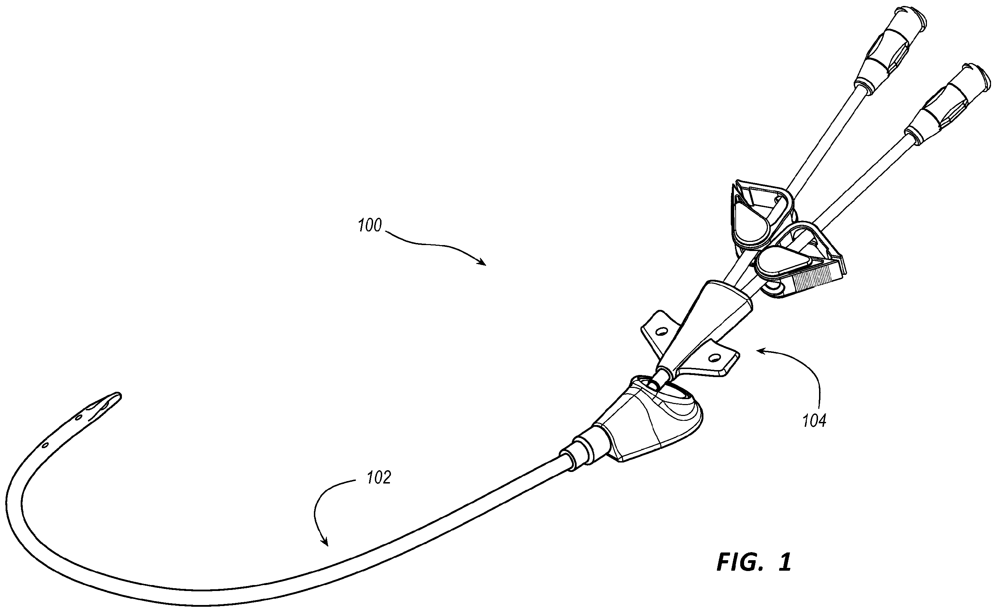

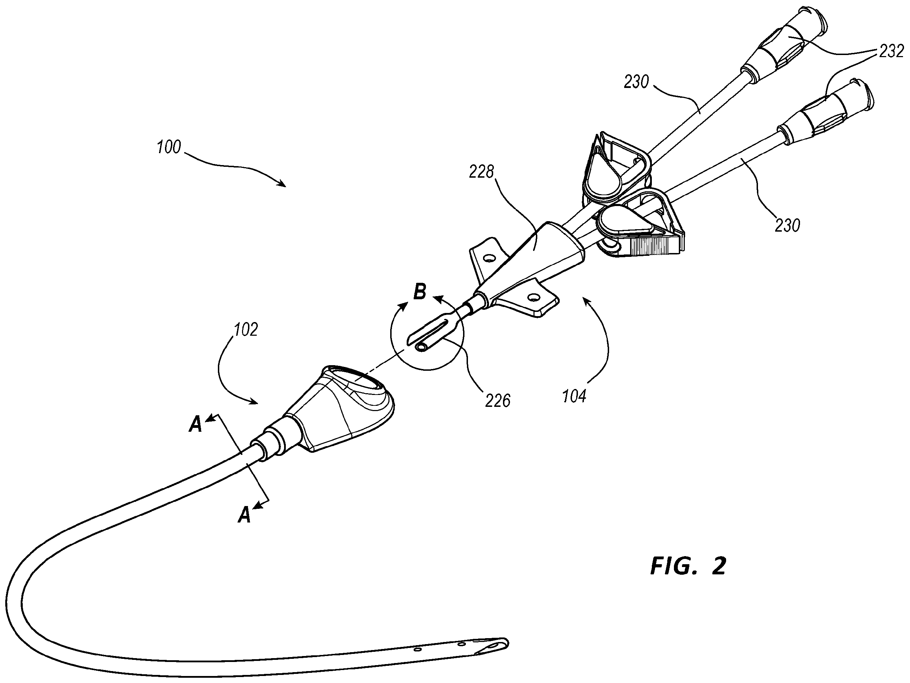

FIG. 1 illustrates a vascular access system 100 including a port 102 and an access device 104 disposed in the port 102 in accordance with some embodiments. FIG. 2 illustrates the vascular access system 100 with the access device 104 separated from the port 102 in accordance with some embodiments.

As shown, the vascular access system 100 includes port 102 and the access device 104. The vascular access system 100 is configured such that the access device 104 can be inserted into the port 102 and removed from the port a number of different times; however, the access device 104 is a single-use access device in that while the port 102 is implanted in a patient, an unused access device 104 should be used each time the access device 104 is inserted into the port 104.

The vascular access system 100 is bi-luminal in that each component of the port 102 and the access device 104 includes two fluid passageways therethrough. Notably, the vascular access system 100 is configured such that the two fluid passageways of the port 102 and the two fluid passageways of the access device 104 fluidly connect upon a single insertion of the access device 104 into the port 102 to form two fluid passageways through the vascular access system 100. A first fluid passageway of the two fluid passageways is configured as an arterial lumen to convey blood away from a heart of a patient. A second fluid passageway of the two fluid passageways is configured as a venous lumen to convey blood to the heart of the patient.

The vascular access system 100 is configured for continuous flow at a flow rate of at least about 300 to 500 mL/min, which is required for hemodialysis.

Additional description for the vascular access system 100 is set forth below with respect to the port 102 and the access device 104.

### Ports

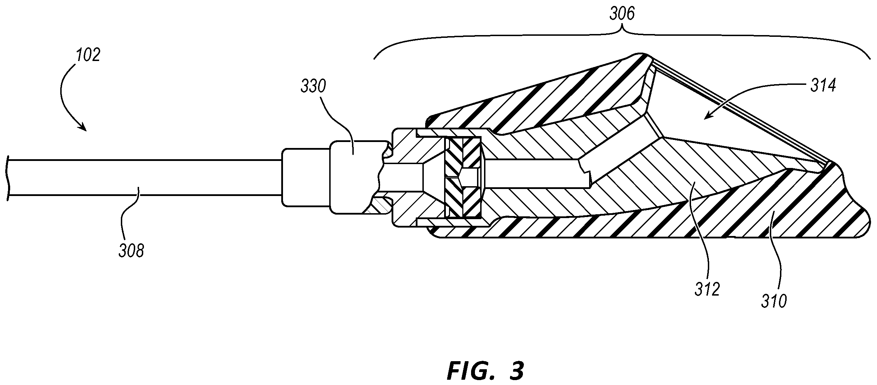

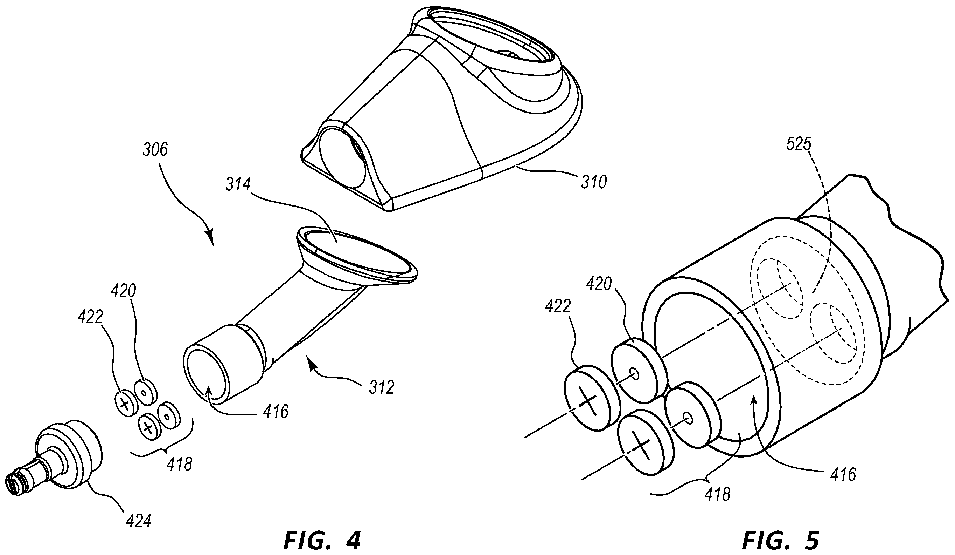

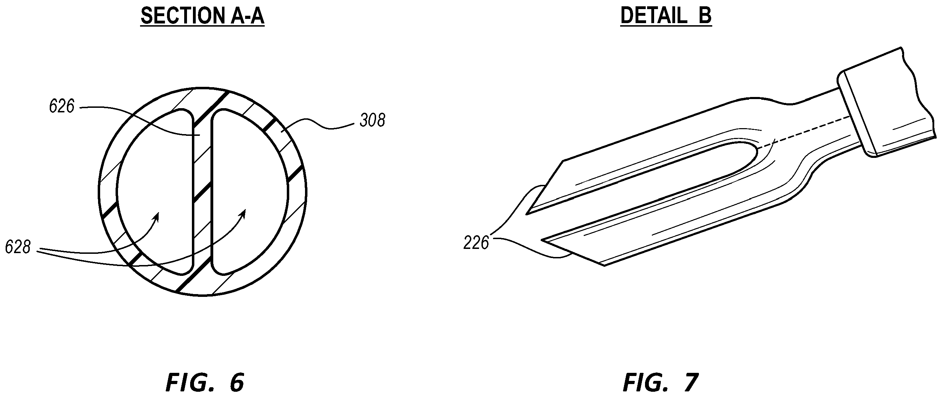

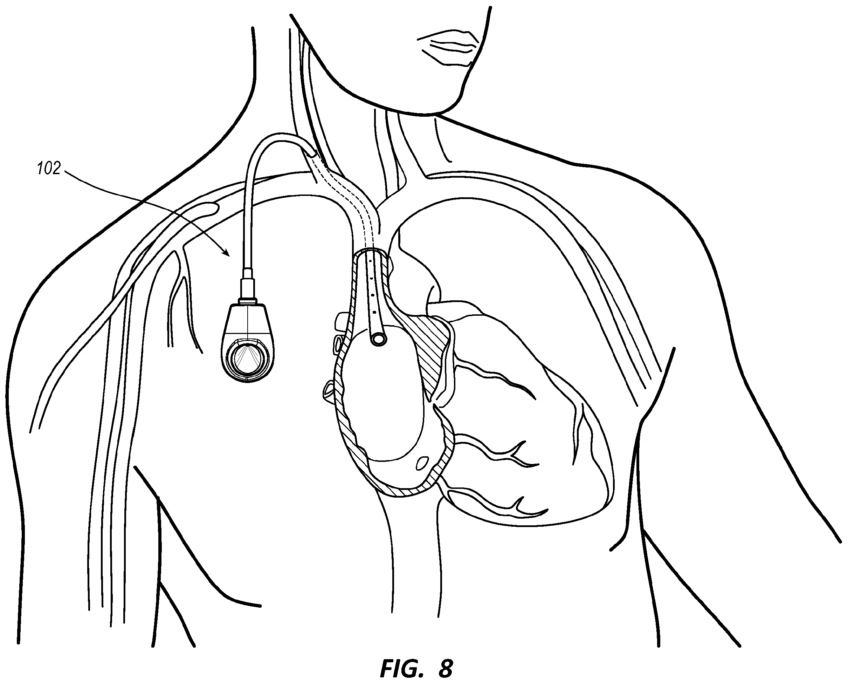

FIG. 3 illustrates a cross-section of a port hub 306 of the port 102 in accordance with some embodiments. FIG. 4 illustrates an exploded view of the port hub 306 in accordance with some embodiments. FIG. 5 illustrates a close-up view of a distal-end portion of the port hub in accordance with some embodiments. FIG. 6 illustrates a cross-section of a catheter tube 308 of the port in accordance with some embodiments. FIG. 8 illustrates the port 102 implanted in a patient in accordance with some embodiments.

As shown, the port 102 includes the port hub 306 and the catheter tube 308.

The port hub 306 includes a body 310 of a medically acceptable polymer having a relatively low durometer such as silicone. The body 310 of such a polymer provides patient comfort when the port 102 is implanted under the subdermis of a patient.

The port hub 306 includes an access funnel 312 disposed in the body 310 configured for a shallow angle of approach or insertion of the access device 104 of less than about 30° between the patient and the pair of cannulas 226 of the access device 104. (*See* FIG. 7 for the pair of cannulas 226 of the access device 104.) A relatively wide opening 314 in a proximal-end portion of the access funnel 312 is configured to catch the pair of cannulas 226 and direct the pair of cannulas 226 into a relatively narrow passageway of the access funnel 312 toward a distal-end portion 416 of the access funnel 312. The access funnel 312 can be titanium or an alloy thereof, which provides the access funnel 312 durability for the number of different times the access device 104 is anticipated to be inserted into the port 102.

The port hub 306 includes a pair of valves 418 disposed in the distal-end portion of the access funnel 312 configured to open when the pair of cannulas 226 of the single-use access device 104 is inserted into the pair of valves 481. Each valve of the pair of valves 418 can include two septums (e.g., silicone septums) sandwiched together such as holed septum 420 and slitted septum 422.

The port hub 306 includes a bifurcated stem 424 extending from the port hub 306 configured to insert into the pair of catheter-tube lumens 628. (*See* FIG. 6 for the pair of catheter-tube lumens 628 of the catheter tube 308.) While not shown, a septum proximally extending from the bifurcated stem 424 in accordance with the bifurcation of the bifurcated stem 424 abuts a septum 525 of the port hub 306 defining a pair of port-hub lumens configured to direct fluid flow from the pair of cannula lumens (*see* description set forth below) of the pair of cannulas 226 into the pair of catheter-tube lumens 628.

The catheter tube 308 includes a septum 626 defining a pair of catheter-tube 628 lumens fluidly coupled to the pair of port-hub lumens when the catheter tube 308 is disposed over the bifurcated stem 424. The catheter tube 308 can have a suitable length for tunneling the catheter tube 308 from a subclavicular access site to a right atrium of a heart. The catheter tube 308 can be a radiopaque polyurethane catheter tube of less than about 10 Fr such as 9 Fr or a catheter size in between (e.g., 9.6 Fr).

The port 102 further includes a catheter lock 330 configured to lock the catheter tube 308 onto the bifurcated stem 424.

### Access devices

FIG. 2 illustrates the vascular access system 100 with the access device 104 separated from the port 102 in accordance with some embodiments. FIG. 7 illustrates a detailed view of a pair of cannulas 226 of the access device 104 in accordance with some embodiments.

As shown, the access device 104 includes the pair of cannulas 226, a bifurcated hub 228, a pair of extension legs 230, and a pair of Luer connectors 232 operably connected in the foregoing order.

The pair of cannulas 226 define a pair of cannula lumens. As set forth above, the pair of cannulas 226 is configured to simultaneously insert into the access funnel 312 of the port hub 306 and fluidly couple the pair of cannula lumens to the pair of catheter-tube lumens when the pair of cannulas 226 is fully seated in the port hub 306. When the port 102 is implanted under the subdermis of a patient, the pair of cannulas 226 is also configured to pierce skin of the patient before insertion into the access funnel 312. Accordingly, each cannula of the pair of cannulas 226 is configured with a beveled tip to pierce the skin of the patient. The pair of cannulas 226 can be a metal or an alloy such as stainless steel, which provides the pair of cannulas 226 sufficient rigidity to pierce the skin of the patient and insert into the access funnel 312.

The bifurcated hub 228 includes a pair of access device-hub lumens fluidly coupled to the pair of cannula lumens of the pair of cannulas 226.

The pair of extension legs 230 define a pair of extension-leg lumens fluidly coupled to the pair of access device-hub lumens of the bifurcated hub 228.

The pair of Luer connectors 232 define a pair of Luer-connector lumens fluidly coupled to the pair of extension-leg lumens of the pair of extension legs 230.

### Methods

A method of vascular access system includes a step of locating an access site and identifying an implanted port such as the port 102 implanted in a patient at the access site by palpation of the patient. The method also includes a step of cleaning and disinfecting the access site with an antiseptic (e.g., iodopovidone) for skin disinfection. The method also includes a step of preparing the access device 104 including priming the access device 104 with saline. The method also includes a step of stabilizing the implanted port 102 with a non-dominant, sterile-gloved hand. The method also includes a step of simultaneously inserting the pair of cannulas 226 into the access funnel 312 until the pair of cannulas 226 is fully seated in the port hub 306, thereby fluidly coupling the pair of cannula lumens to the pair of catheter-tube lumens 628. Inserting the pair of cannulas 226 into the access funnel 312 includes piercing skin of the patient and inserting the pair of cannulas 226 into the access funnel 312 with a shallow angle of approach of less than about 30° between the patient and the pair of cannulas 226. The method also includes a step of aspirating the vascular access system 100 for blood flow, as well as a step of securely dressing the access site with a wound dressing.

The vascular access system 100 leverages benefits of a fully subcutaneous port, which allows patients the ability to bathe or shower without inconveniencing the patients or adversely affecting their ports. Indeed, no portion of the port 102 is subject to exposure except during a hemodialysis procedure when the access device 104 is being used. Being a fully subcutaneous port improves cosmesis and obviates routine maintenance otherwise required to avoid clots and infection.

The vascular access system 100 also leverages benefits of the access device 104 looking and operating much like a standard hemodialysis catheter once the access device 104 is seated in the port 102. For example, the pair of extension legs 230 should appear familiar to clinicians and take little to no training to connect to other hemodialysis equipment.

## Claims

1. A vascular access system (100), comprising:
a port (102) configured for subdermal implantation, the port (102) including:
a port hub (306) having an access funnel (312) and a septum (525) defining a pair of port-hub lumens; and
a catheter tube (308) having a septum (626) defining a pair of catheter-tube lumens (628) fluidly coupled to the pair of port-hub lumens; and
a single-use access device (104), **characterised in that** the access device (104) includes:
a bifurcated hub (228) including a pair of access device-hub lumens; and
a pair of cannulas (226) defining a pair of cannula lumens fluidly coupled to the pair of access device-hub lumens, the pair of cannulas (226) configured to simultaneously insert into the access funnel (312) and fluidly couple the pair of cannula lumens to the pair of catheter-tube lumens (628) when the pair of cannulas (226) is fully seated in the port hub (306).

2. The vascular access system (100) of claim 1, the pair of cannulas (226) configured to pierce skin of a patient before inserting the pair of cannulas (226) into the access funnel (312) of the port (102) when implanted under the skin.

3. The vascular access system (100) of either claim 1 or 2, the single-use access device (104) including a pair of extension legs (230) defining a pair of extension-leg lumens fluidly coupled to the pair of access device-hub lumens.

4. The vascular access system (100) of any claim of claims 1-3, the port hub (306) having a pair of valves (418) in a distal-end portion of the access funnel (312) configured to open when the pair of cannulas (226) is inserted into the pair of valves (418).

5. The vascular access system (100) of any claim of claims 1-4, the port hub (306) having a bifurcated stem (424) extending from the port hub (306) configured to insert into the pair of catheter-tube lumens(628).

6. The vascular access system (100) of claim 5, wherein the port (102) preferably including a catheter lock (330) over the catheter tube (308) over the bifurcated stem (424), the catheter lock (330) configured to lock the catheter tube (308) onto the bifurcated stem (424).

7. The vascular access system (100) of any claim of claims 1-6, the vascular access system (100) configured for continuous flow at a flow rate of at least 300 to 500 mL/min.

8. The vascular access system (100) of any claim of claims 1-7, wherein the catheter tube (308) is a radiopaque polyurethane catheter tube (308).

9. The vascular access system (100) of any claim of claims 1-8, wherein the access funnel (312) is titanium or an alloy thereof, the access funnel (312) configured for a relatively low-angle approach by a single-use access device (104).

10. The vascular access system (100) of any claim of claims 1-9, wherein the access funnel (312) is disposed within a silicone body (310) of the port hub (306).

11. The vascular access system (100) of claim 3, further comprising a pair of Luer connectors (232), each Luer connector (232) connected to an extension leg (230) of the pair of extension legs (230).

## Patentansprüche

1. Vaskuläres Zugangssystem (100), umfassend:
einen Anschluss (102), der zur subdermalen Implantation ausgebildet ist, der Anschluss (102) einschließend:
eine Anschlussnabe (306), die einen Zugangstrichter (312) und ein Septum (525), das ein Paar von Anschlussnabenlumen definiert, aufweist; und
einen Katheterschlauch (308), der ein Septum (626) aufweist, das ein Paar von Katheterschlauchlumen (628) definiert, das fluidisch mit dem Paar von Anschlussnabenlumen gekoppelt ist; und
eine Einweg-Zugangsvorrichtung (104), **dadurch gekennzeichnet, dass** die Zugangsvorrichtung (104) einschließt:
eine gegabelte Nabe (228), die ein Paar von Zugangsvorrichtungsnabenlumen einschließt; und
ein Paar von Kanülen (226), das ein Paar von Kanülenlumen definiert, das fluidisch mit dem Paar von Zugangsvorrichtungsnabenlumen gekoppelt ist, wobei das Paar von Kanülen (226) dazu ausgebildet ist, in den Zugangstrichter (312) eingeführt zu werden und gleichzeitig das Paar von Kanülenlumen fluidisch mit dem Paar von Katheterschlauchlumen (628) zu koppeln, wenn das Paar von Kanülen (226) vollständig in der Anschlussnabe (306) sitzt.

2. Vaskuläres Zugangssystem (100) nach Anspruch 1, wobei das Paar von Kanülen (226) dazu ausgebildet ist, die Haut eines Patienten zu durchstechen, bevor das Paar von Kanülen (226) in den Zugangstrichter (312) des Anschlusses (102) eingeführt wird, wenn dieser unter der Haut implantiert ist.

3. Vaskuläres Zugangssystem (100) nach entweder Anspruch 1 oder 2, wobei die Einweg-Zugangsvorrichtung (104) ein Paar von Verlängerungsschenkeln (230) einschließt, das ein Paar von Verlängerungsschenkellumen definiert, das fluidisch mit dem Paar von Zugangsvorrichtungsnabenlumen gekoppelt ist.

4. Vaskuläres Zugangssystem (100) nach einem Anspruch der Ansprüche 1-3, wobei die Anschlussnabe (306) in einem distalen Endabschnitt des Zugangstrichters (312) ein Paar von Ventilen (418) aufweist, das dazu ausgebildet ist, sich zu öffnen, wenn das Paar von Kanülen (226) in das Paar von Ventilen (418) eingeführt wird.

5. Vaskuläres Zugangssystem (100) nach einem Anspruch der Ansprüche 1-4, wobei die Anschlussnabe (306) einen gegabelten Schaft (424) aufweist, der sich von der Anschlussnabe (306) erstreckt und dazu ausgebildet ist, in das Paar von Katheterschlauchlumen (628) eingeführt zu werden.

6. Vaskuläres Zugangssystem (100) nach Anspruch 5, wobei der Anschluss (102) über dem Katheterschlauch (308) über dem gegabelten Schaft (424) vorzugsweise eine Katheterverriegelung (330) einschließt, wobei die Katheterverriegelung (330) dazu ausgebildet ist, den Katheterschlauch (308) auf dem gegabelten Schaft (424) zu verriegeln.

7. Vaskuläres Zugangssystem (100) nach einem Anspruch der Ansprüche 1-6, wobei das vaskuläre Zugangssystem (100) für einen kontinuierlichen Fluss mit einer Flussrate von mindestens 300 bis 500 ml/min ausgebildet ist.

8. Vaskuläres Zugangssystem (100) nach einem Anspruch der Ansprüche 1-7, wobei der Katheterschlauch (308) ein röntgendichter Polyurethan-Katheterschlauch (308) ist.

9. Vaskuläres Zugangssystem (100) nach einem Anspruch der Ansprüche 1-8, wobei der Zugangstrichter (312) Titan oder eine Legierung davon ist, wobei der Zugangstrichter (312) für eine Annäherung mit einem relativ flachen Winkel durch eine Einweg-Zugangsvorrichtung (104) ausgebildet ist.

10. Vaskuläres Zugangssystem (100) nach einem Anspruch der Ansprüche 1-9, wobei der Zugangstrichter (312) innerhalb eines Silikonkörpers (310) der Anschlussnabe (306) angeordnet ist.

11. Vaskuläres Zugangssystem (100) nach Anspruch 3, weiter umfassend ein Paar von Luer-Verbindern (232), wobei jeder Luer-Verbinder (232) mit einem Verlängerungsschenkel (230) des Paares von Verlängerungsschenkeln (230) verbunden ist.

## Revendications

1. Système (100) d'accès vasculaire, comprenant :
une chambre implantable (102), configurée pour une implantation sous-dermique, la chambre implantable (102) incluant :
une partie de base (306) de chambre, présentant un entonnoir d'accès (312) et un septum (525) définissant une paire de lumières de partie de base de chambre ; et,
un tube de cathéter (308), présentant un septum (626) définissant une paire de lumières (628) de tube de cathéter reliées fluidiquement à la paire de lumières de partie de base de chambre ; et
un dispositif d'accès (104) à usage unique, **caractérisé en ce que** le dispositif d'accès (104) inclut :
une partie de base bifurquée (228), incluant une paire de lumières de partie de base de dispositif d'accès ; et,
une paire de canules (226), définissant une paire de lumières de canule reliées fluidiquement à la paire de lumières de partie de base de dispositif d'accès, la paire de canules (226) étant configurée pour s'insérer simultanément dans l'entonnoir d'accès (312) et relier fluidiquement la paire de lumières de canule à la paire de lumières (628) de tube de cathéter lorsque la paire de canules (226) est entièrement logée dans la partie de base (306) de chambre.

2. Système (100) d'accès vasculaire selon la revendication 1, la paire de canules (226) étant configurée pour percer la peau d'un patient avant d'insérer la paire de canules (226) dans l'entonnoir d'accès (312) de la chambre implantable (102) lorsque cette dernière est implantée sous la peau.

3. Système (100) d'accès vasculaire selon l'une ou l'autre des revendications 1 ou 2, le dispositif d'accès (104) à usage unique incluant une paire de branches d'extension (230) définissant une paire de lumières de branche d'extension reliées fluidiquement à la paire de lumières de partie de base de dispositif d'accès.

4. Système (100) d'accès vasculaire selon une revendication quelconque parmi les revendications 1 à 3, la partie de base (306) de chambre présentant une paire de valves (418) dans une portion d'extrémité distale de l'entonnoir d'accès (312), configurée pour s'ouvrir lorsque la paire de canules (226) est insérée dans la paire de valves (418).

5. Système (100) d'accès vasculaire selon une revendication quelconque parmi les revendications 1 à 4, la partie de base (306) de chambre présentant une tige bifurquée (424) s'étendant depuis la partie de base (306) de chambre, configurée pour s'insérer dans la paire de lumières (628) de tube de cathéter.

6. Système (100) d'accès vasculaire selon la revendication 5, dans lequel la chambre implantable (102) inclut de préférence un dispositif de verrouillage de cathéter (330) sur le tube de cathéter (308) sur la tige bifurquée (424), le dispositif de verrouillage de cathéter (330) étant configuré pour verrouiller le tube de cathéter (308) sur la tige bifurquée (424).

7. Système (100) d'accès vasculaire selon une revendication quelconque parmi les revendications 1 à 6, le système (100) d'accès vasculaire étant configuré pour un flux continu à un débit d'au moins 300 à 500 mL/min.

8. Système (100) d'accès vasculaire selon une revendication quelconque parmi les revendications 1 à 7, dans lequel le tube de cathéter (308) est un tube de cathéter (308) en polyuréthane radio-opaque.

9. Système (100) d'accès vasculaire selon une revendication quelconque parmi les revendications 1 à 8, dans lequel l'entonnoir d'accès (312) est en titane ou un alliage de celui-ci, l'entonnoir d'accès (312) étant configuré pour une approche à un angle relativement faible par un dispositif d'accès (104) à usage unique.

10. Système (100) d'accès vasculaire selon une revendication quelconque parmi les revendications 1 à 9, dans lequel l'entonnoir d'accès (312) est disposé à l'intérieur d'un corps en silicone (310) de la partie de base (306) de chambre.

11. Système (100) d'accès vasculaire selon la revendication 3, comprenant en outre une paire de connecteurs Luer (232), chaque connecteur Luer (232) étant relié à une branche d'extension (230) de la paire de branches d'extension (230).
